# EUROPEAN PATENT SPECIFICATION

(11) **EP 2 049 122 B1**
(45) Date of publication and mention of the grant of the patent: **08.10.2014**
(21) Application number: 07763798.1
(22) Date of filing: 03.08.2007
(51) Int. Cl.: A61K 31/568, A61K 31/58, A61K 31/704, A61K 31/7048, A61P 1/00, A61P 9/00, A61P 11/00, A61P 17/02, A61P 17/04, A61P 19/02, A61P 19/04, A61P 27/02, A61P 37/00, A61K 45/06

(54) **COMPOSITIONS FOR INHIBITING ANGIOGENESIS**
ZUSAMMENSETZUNGEN ZUR HEMMUNG DER ANGIOGENESE
COMPOSITIONS D'INHIBITION DE L'ANGIOGENÈSE

(30) Priority: 03.08.2006 AU 2006904195
(43) Date of publication of application: 22.04.2009
(73) Proprietor: Oncology Research International Limited, Perth, WA 6000 (AU)
(72) Inventor: STORY, Michael John, Carrickalinga, South Australia 5204 (AU); WAYTE, Kenneth Michael, Ocean Reef, Western Australia 6027 (AU)
(74) Representative: Kudlek & Grunert Patentanwälte
(86) International application number: PCT/AU2007/001092
(87) International publication number: WO 2008/014564

(56) References cited:
- WO-A1-03/035092
- WO-A1-2005/060977
- WO-A1-2005/060977
- WO-A1-2005/116042
- WO-A2-2006/053184
- WO-A2-2007/003957
- CN-A- 1 237 583
- CN-A- 1 237 583
- JP-A- 3 271 224
- JP-A- 8 040 914
- US-A- 5 919 770
- US-A- 5 985 330
- US-A1- 2002 045 202
- US-A1- 2003 203 856
- US-A1- 2005 288 239
- US-B1- 6 168 795
- US-B1- 6 395 279
- US-B1- 6 444 233
- LEE MEI-SZE ET AL: "Effects of polyphyllin D, a steroidal saponin in Paris polyphylla, in growth inhibition of human breast cancer cells and in xenograft.", CANCER BIOLOGY & THERAPY NOV 2005 LNKD- PUBMED:16258257, vol. 4, no. 11, November 2005 (2005-11), pages 1248-1254, XP002629731, ISSN: 1538-4047
- ZHANG HONG-JIE ET AL: "Bioactive constituents from Asparagus cochinchinensis.", JOURNAL OF NATURAL PRODUCTS FEB 2004 LNKD- PUBMED:14987058, vol. 67, no. 2, February 2004 (2004-02), pages 194-200, XP002629732, ISSN: 0163-3864
- LIU MING-JIE ET AL: "The mitotic-arresting and apoptosis-inducing effects of diosgenyl saponins on human leukemia cell lines", BIOLOGICAL & PHARMACEUTICAL BULLETIN, vol. 27, no. 7, July 2004 (2004-07), pages 1059-1065, XP002629733, ISSN: 0918-6158
- RONG-TSUN WU ET AL: "Formosanin-C, an immunomodulator with antitumor activity", INTERNATIONAL JOURNAL OF IMMUNOPHARMACOLOGY, ELMSFORD,NY, US, vol. 12, no. 7, 1 January 1990 (1990-01-01), pages 777-786, XP025483992, ISSN: 0192-0561, DOI: DOI:10.1016/0192-0561(90)90042-L [retrieved on 1990-01-01]
- BARTHOMEUF C. ET AL.: 'Inhibition of HUVEC Tubulogenesis by hederacolchiside-A1 is Associated with Plasma Membrane Cholesterol Sequestration and Activation of the Ha-Ras/MEK/ERK Cascae' CANCER CHEMOTHERAPY AND PHARMACOLOGY vol. 54, 2004, pages 432 - 440, XP008103282
- TONG Y. ET AL.: 'Philinopside A, A Novel Marine-Derived Compound Possessing Dual Anti-Angiogenic and Anti-Tumor Effects' INTERNATIONAL JOURNAL OF CANCER vol. 114, 2005, pages 843 - 853, XP008103280

## Description

### Field of the Invention

The present invention relates to a steroid saponin selected from the group consisting of deltonin, dioscin and prosapogenin A for use in inhibiting angiogenesis in a human or animal subject, wherein the angiogenesis is angiogenesis associated with a disease mentioned in claim 1.

### Background of the Invention

Angiogenesis is the process of forming new blood vessels from pre-existing blood vessels by the growth and migration of endothelial cells in a process called "sprouting".

The growth of endothelial cells is a critical step in the angiogenic process. Organs have well-marked boundaries defined by surrounding acellular structures called basement membranes which are made up of a fabric of extracellular matrix (ECM) proteins, predominantly laminins, type IV collagen and protoglycans. Angiogenesis commences with the erosion of the basement membrane surrounding endothelial cells which line the lumen of blood vessels. Erosion of the basement membrane is triggered by enzymes released by endothelial cells and leukocytes. The endothelial cells then migrate through the eroded basement membrane when induced by angiogenic stimulants. The migrating cells form a "sprout" off the parent blood vessel. The migrating endothelial cells proliferate, and the sprouts merge to form capillary loops, thus forming a new blood vessel.

The control of angiogenesis is a highly regulated process involving the actions of a number of angiogenic stimulators and inhibitors. Both controlled and uncontrolled angiogenesis are thought to proceed in a similar manner.

Under normal physiological conditions, humans and animals only undergo angiogenesis in very specific restricted situations. For example, angiogenesis is only normally observed in wound healing, foetal and embryonic development, and formation of the corpus luteum, endometrium and placenta.

However, uncontrolled or undesired angiogenesis is associated with many diseases and conditions. The induction of angiogenesis is a hallmark of cancer, characterised by the spreading of tumour cells throughout the body. The process whereby tumour cells migrate from a primary site to a secondary site is called "metastasis" which is the fundamental difference between a benign and malignant growth and represents the major clinical problem of cancer. Unfortunately, over 50% of solid tumours have metastasised at the time of diagnosis.

The evidence for the role of angiogenesis in tumour growth is extensive and it is generally accepted that the growth of any solid tumour is critically dependent upon this process. Angiogenesis plays a critical role in two stages of tumour development. Firstly, angiogenesis is required for a tumour mass to grow beyond a size of a few millimetres. Without the formation of new vasculature, the cells in the tumour mass will not receive sufficient blood supply to develop beyond this small size. However, once vascularization of the tumour commences, the tumour mass may then expand.

Vascularization of the tumour also plays a significant role in the development of secondary tumours. Vascularization of the tumour allows tumour cells to enter the blood stream and to circulate throughout the body. After the tumour cells have left the primary site and settled into a secondary (metastatic) site, further angiogenesis then allows the secondary tumour mass to grow and expand. Therefore, prevention of angiogenesis may not only lead to a reduction in the growth of a tumour at its primary site; but the prevention of angiogenesis may also inhibit or reduce the migration of tumour cells from the primary site to other parts of the body (metastasis).

In addition to the formation of tumours, there are also various diseases and conditions induced by angiogenesis or associated with uncontrolled or undesired angiogenesis. These include diabetic retinopathy, retrolental fibroplasia, neovascular glaucoma, psoriasis, angiofibroma, immune and non-immune inflammation (including rheumatic arthritis), propagation of capillary vessels in arteriosclerosis plaques, angioma and Kaposi's sarcoma. Angiogenesis can also occur in a rheumatoid joint, hastening joint destruction by allowing an influx of leukocytes with subsequent release of inflammatory mediators.

Angiogenesis also plays a pivotal role in the cornea and retina of patients with certain ocular disorders, for example ocular neovascular disease. This disease is characterized by invasion of new blood vessels into the structures of the eye such as the retina or cornea. It is the most common cause of blindness and is associated with a large number of diseases of the eye. In age-related macular degeneration, the associated visual problems are caused by an ingrowth of choroidal capillaries through defects in Bruch's membrane with proliferation of fibrovascular tissue beneath the retinal pigment epithelium.

Chronic inflammation may also involve pathological angiogenesis. Disease states such as ulcerative colitis and Crohn's disease show histological changes with the ingrowth of new blood vessels into the inflamed tissues. Another pathological role associated with angiogenesis is found in atherosclerosis. The plaques formed within the lumen of blood vessels have been shown to have angiogenic stimulatory activity.

Angiogenesis is also involved in reproduction and wound healing. In reproduction, angiogenesis is an important step in ovulation and also in implantation of the blastula after fertilization. Prevention of angiogenesis may be used to induce amenorrhea, to block ovulation, or to prevent implantation by the blastula. In wound healing, excessive repair or fibroplasia can be a detrimental side effect of surgical procedures and may be caused or exacerbated by angiogenesis. Adhesions are a frequent complication of surgery and lead to problems such as small bowel obstruction.

The current treatment of diseases involving uncontrolled or undesired angiogenesis is inadequate. Accordingly, there is a need for new methods and compositions that inhibit uncontrolled or undesired angiogenesis.

The present invention arises from the determination that steroid saponins have anti-angiogenic capacity, and relates to a steroid saponin for use in inhibiting angiogenesis in a human or animal subject.

A reference herein to a patent document or other matter which is given as prior art is not to be taken as an admission that the document or matter was known or that the information it contains was part of the common general knowledge as at the priority date of any of the claims.

The use of specific steroid saponins for the treatment of cancer is known from WO 2005/060977 A1, CN 1237583 A, JP 8 040914 A and JP 3271224 A and is also disclosed in Lee Mei-Sze et al.: "Effects of polyphyllin D, a steroidal saponin in Paris polyphylla, in growth inhibition of human breast cancer cells and in xenograft", Cancer Biology & Therapy, vol. 4, no. 11, 2005, pages 1248-1254, Zhang Hong-Jie et al.: "Bioactive constituents from Asparagus cochinchinensis", Journal of Natural Products, vol. 67, no. 2, 2004, pages 194-200, Liu Ming-Jie et al.: "The mitotic-arresting and apoptosis-inducing effects of diosgenyl saponins on human leukemia cell lines", Biological & Pharmaceutical Bulletin, vol. 27, no. 7, 2004, pages 1059-1065, and Rong-Tsun Wu et al.: "Formosanin-C, an immunomodulator with antitumor activity", International Journal of Immunopharmacology, vol. 12, no. 7, 1990, pages 777-786.

WO2005/060977 discloses the use of various steroid saponins as inhibitors of Core 2GlcNAc-T for treating diseases such as inflammatory diseases, atherosclerosis, diabetic retinopathy etc (page 7, line 5-13). Compounds include both furostanol and spirostanol saponins which are described by 11 different Markush formulae (p. 29, I. 15-24, page 8-10, claims 1-25). Diosgenin and deltonin are mentioned in a long list of so termed "preferred" compounds (p. 23, I. 4-16). However they do not belong to the so termed "particularly preferred compounds", namely trigeoniside IVa, Glycoside F, Shatavarin I, compound 3 and pardarinoside C (see page 16, line 14-page 17, line 5). The experiments only show an in vitro activity in inhibiting GlcNAc-T for the so termed preferred compounds, trigeonoside IVa, glycoside F and shatavarin (see page 41). A therapeutic activity in treating diabetic retinopathy has been demonstrated only for fenukreed seeds extract (page 44, line 22-24).

### Summary of the Invention

The present invention arises out of studies into the ability of steroid saponins to inhibit angiogenesis. In particular, it has been found that steroid saponins have the ability to inhibit angiogenesis in a number of *in vivo* and *ex vivo* model systems. This finding indicates that steroid saponins have significant anti-angiogenic capacity.

Accordingly, the present invention provides a steroid saponin for use in inhibiting angiogenesis in a human or animal subject according to claim 1, which is selected from the group consisting of deltonin, dioscin, prosapogenin A.

Various terms that will be used throughout the specification have meanings that will be well understood by a skilled addressee. However, for ease of reference, some of these terms will now be defined.

The term "glycoside" as used throughout the specification is to be understood to mean a compound that contains a saccharide (sugar) moiety (monosaccharide, disaccharide or polysaccharide), linked to a triterpene or steroid or steroid alkaloid aglycone (non-saccharide) component. In most circumstances, the saccharide (sugar) moiety is linked to the C-3 position of the aglycone, although other linkages are contemplated within the scope of the present invention. For example the furostanol glycosides, which contain a saccharide attached to the C-26 position, and spirostanol glycosides are both sub-classes of the steroid saponins.

The term "saponin" as used throughout the specification is to be understood to mean a glycoside including a saccharide (sugar) attached to the aglycone, generally through the C-3 position of the aglycone.

The term "steroid saponin" as used throughout the specification is to be understood to mean a glycoside including one or more saccharide units (including one or more monosaccharide, disaccharide or polysaccharide units) attached to an aglycone which does not contain a nitrogen atom.

A steroid "aglycone" is also called a "genin" or sapogenin" and the terms may be used interchangeably throughout the specification and all are to be understood to mean the non-saccharide portion of a saponin molecule.

The term "saccharideA-(1→n)-saccharideB" as used throughout the specification is to be understood to mean that saccharideA is linked by its C-1 to the C-n of saccharideB, n being an integer.

For example the polysaccharide with the common name "chacotriose" is α-L-rhamnopyranosyl-(1→2)-[α-L-rhamnopyranosyl-(1→4)]-β-D-glucopyranoside. An abbreviated form of nomencalture in accordance with IUPAC recommendations used herein is Rha 2, [Rha 4], Glc.

The term "subject" as used throughout the specification is to be understood to mean any human or animal subject. In this regard, it will be understood that the present invention includes within its scope veterinary applications. For example, the animal subject may be a mammal, a primate, a livestock animal (eg. a horse, a cow, a sheep, a pig, or a goat), a companion animal (eg. a dog, a cat), a laboratory test animal (eg. a mouse, a rat, a guinea pig, a bird), an animal of veterinary significance, or an animal of economic significance.

The term "treat", and variants thereof as used throughout the specification, is to be understood to mean therapeutic intervention with an effective amount of a steroid saponin. For example, the term includes within its scope therapeutic intervention to have one or more of the following outcomes: (i) to inhibit or prevent the growth of a primary tumour in a subject, including reducing the growth of the primary tumour after resection; (ii) inhibit or prevent the growth and formation of one or more secondary tumours in a subject; (iii) inhibit or prevent angiogenesis in a subject; (iv) inhibit endothelial cell proliferation and/or migration in a subject; (v) improve the life expectancy of the subject as compared to the untreated state; and (vi) improve the quality of life the subject as compared to the untreated state.

The term "inhibit" as used throughout the specification is to be understood to mean a reduction in the progress of a process, including any one or more of the start, rate, probability, continuation or termination of a process.

The term "angiogenesis" as used throughout the specification is to be understood to mean the generation of new blood vessels ("neovascularization"), for example into a tissue or organ.

The term "biological system" as used throughout the specification is to be understood to mean any multi-cellular system and includes isolated groups of cells to whole organisms. For example, the biological system may be cells in tissue culture, a tissue or organ, or an entire human subject, such as a human subject suffering the effects of undesired or uncontrolled angiogenesis, or a disease or condition associated with uncontrolled or undesired angiogenesis.

The term "anti-angiogenic agent" as used throughout the specification is to be understood to mean an agent that has the capacity to inhibit angiogenesis in a biological system.

### Brief Description of the Figures

Figure 1 shows the effect of increasing concentration of deltonin on vessel growth from aortic explants.
Figure 2 shows a representative example of the effect of DMSO alone on aortic explants using light microscopy or Fitc-BS-1 lectin staining.
Figure 3 shows a representative example of the effect of 10 µM deltonin on aortic explants using light microscopy or Fitc-BS-1 lectin staining.
Figure 4 shows the effect of dioscin, deltonin, trillin, prosapogenin A and sorafenib on CD31 staining of blood vessels in angiosponges, as an indicator of the effect of these compounds on the average number of blood vessels induced.
Figure 5 shows the effect of dioscin, deltonin, trillin, prosapogenin A and sorafenib on the percent induction of angiogenesis by bFGF in angiosponges.
Figure 6 shows the effect of dioscin, deltonin, trillin, prosapogenin A and sorafenib on blood volume in angiochambers.
Figure 7 shows the effect of dioscin, deltonin, trillin, prosapogenin A and sorafenib on induction of angiogenesis by bFGF in angiochambers.

### General Description of the Invention

As mentioned above, the present invention provides a steroid saponin for use in inhibiting angiogenesis in a human or animal subject according to claim 1.

According to the invention, the biological system is a human or animal subject. In one specific embodiment, the biological system is a human or animal subject in which angiogenesis is associated with a disease or condition that is due to undesired or uncontrolled angiogenesis.

The biological system is a human or animal subject suffering from, or susceptible to, angiogenesis associated with the formation or expansion of angiofibroma, corneal neovascularisation, retinal/choroidal neovascularization, diabetic retinopathy, age-related macular degeneration, arteriovenous malformations, arthritis, rheumatoid arthritis, osteoarthritis, psoriatic arthritis, lupus, connective tissue disorders, Osler-Weber syndrome, atherosclerotic plaques, psoriasis, pyogenic granuloma, retrolental fibroplasias, scleroderma, hemangioma; trachoma, haemophilic joints, hypertrophic scars, diseases or conditions associated with acute or chronic inflammation, diseases or conditions associated with chronic inflammation of the lung including asthma, sarcoidosis, inflammatory bowel diseases, Crohn's disease or ulcerative colitis.

The subject in the various embodiments of the present invention is a human or animal subject. For example, the animal subject may be a mammal, a primate, a livestock animal (eg. a horse, a cow, a sheep, a pig, or a goat), a companion animal (eg. a dog, a cat), a laboratory test animal (eg. a mouse, a rat, a guinea pig, a bird), an animal of veterinary significance, or an animal of economic significance.

In one embodiment, the subject is a human subject.

Saponins are conventionally divided into three major classes: (i) triterpene glycosides; (ii) steroidal glycosides; and (iii) steroidal alkaloid glycosides. They all have in common the attachment of one or more sugar units to the aglycone, generally at the C-3 position. Steroid saponins are generally as described in Hostettmann K and Marston A (2005). Chemistry & pharmacology of natural products: Saponins. Cambridge University Press.

As discussed previously herein, steroid saponins do not contain a nitrogen atom in the aglycone moiety.

It will be appreciated that steroid saponins generally include naturally occurring steroid saponins and non-naturally occurring steroid saponins (ie chemically synthesized steroid saponins). In addition, it will also be appreciated that steroid saponins generally also includes pro-drugs of the steroid saponins, derivatives of steroid saponins, including for example, any esters, ketones, carboxylic acids, salts, substituted forms, halogenated forms or other heteroatom containing forms, unsaturated forms, or any other functional derivative.

The saccharide portion of a steroid saponins generally may include one or more saccharide units, such as a monosaccharide, a disaccharide unit or a polysaccharide unit.

It will also be appreciated that a steroid saponin generally may also include an aglycone with a saccharide attached at one or more positions of the aglycone moiety.

A steroid saponin can include a saccharide attached to a single position of the sapogenin component of the steroid saponin.

As discussed above, the saccharide unit generally may be a monosaccharide, a disaccharide or a polysaccharide. The saccharide may be composed of a suitable monosaccharide, such as D-glucose (Glc), L-rhamnose (Rha), D-galactose (Gal), D-glucuronic acid (GlcA), D-xylose (Xyl), L-arabinose (Ara), D-fucose (Fuc), D-galacturonic acid (GalA). The saccharide unit may also be a substituted sugar, such as an amino sugar, a sulphated sugar, an acylated sugar, a N-acylated sugar, and functional derivatives of any of the aforementioned monosaccharides.

Similarly, a disaccharide generally may be any combination of two monosaccharides, as described above.

The polysaccharides generally may be linear or branched, and include any combination of two or more monosaccharide, including the monosaccharide described previously herein.

Generally, the polysaccharide is composed of 1 to 6 monosaccharide units.

In this regard, and as described previously herein, polysaccharides are generally described in the context of the arrangement of the component monosaccharides.

The saccharide of the steroid saponin generally may be composed of 1 monosaccharide unit. An example of a monosaccharide is glucose with the chemical name β-D-glucopyranoside, which when attached to the aglycone diosgenin via the C-3 position, has the common name of "trillin."

In one embodiment the saccharide of the steroid saponin is composed of 2 monosaccharide units ie a disaccharide. The disaccharide is Rha 2, Glc with the chemical name α-L-rhamnopyranosyl(1→2)-β-D-glucopyranoside, which when attached to the aglycone diosgenin via the C-3 position, has the common name of "prosapogenin A."

In another embodiment the polysaccharide is composed of 3 saccharide units, ie a trisaccharide. According to the invention, chacotrioside is the trisaccharide unit, where the glycosyl group of three saccharides has two rhamnose units linked to a glucose unit which in turn is linked via a glycosidal linkage to the C-3 position of a sapogenin. Chacotriose is α-L-rhamnopyranosyl-(1→2)-[α-L-rhamnopyranosyl-(1→4)]-β-D-glucopyranoside. An abbreviated form of nomencalture in accordance with IUPAC recommendations used herein is Rha 2, [Rha 4], Glc.

In another embodiment, the steroid saponin is dioscin, where dioscin is the sapogenin diosgenin linked through the C-3 position to chacotriose (Rha 2, [Rha 4], Glc).

In another embodiment, the steroid saponin is deltonin where deltonin is the sapogenin diosgenin linked through the C-3 position to Rha 2, [Glc 4], Glc.

Similarly solatrioside is a glycosyl group of three saccharides, having one rhamnose unit and a non-rhamnose saccharide unit, each linked to a third saccharide unit, which is in turn linked via a glycosidal linkage to the C-3 position of a sapogenin.

An example of a tetrasaccharide is [Rha 4, Rha 4], Rha 2, Glc with the chemical name [α-L-rhamnopyranosyl(1→4)-α-L-rhamnopyranosyl(1→4)]-α-L-rhamnopyranosyl(1→4)-β-D-glucopyranoside, which when attached to the aglycone diosgenin via the C-3 position has the common name of "asperin."

Another example of a tetrasaccharide is Glc 4, [Xyl 3], Rha 2, Ara, with the chemical name β-D-glucopyranosyl(1→4)-[β-D-xylopyranosyl-(1→3)]-α-L-rhamnopyrano-syl(1→2)-α-L-arabinoside.

As discussed previously steroid saponins do not contain a nitrogen atom in the aglycone moiety.

Generally, a steroid saponin may be based on a sapogenin with the chemical Formula I or II as follows: 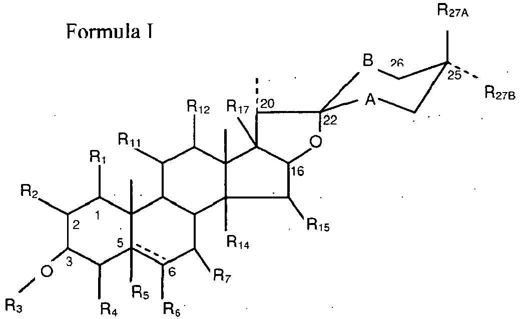 wherein
R₁, R₂, R₄, R₆, R₇, R₁₁, R₁₂, R₁₄, R₁₅ and R₁₇ are independently H, OH, =O, pharmacologically acceptable ester groups or pharmacologically acceptable ether groups;
R₅ is H when C-5,C-6 is a single bond, and nothing when C-5,C-6 is a double bond;
A is either O concurrently with B being CH₂, or B is O concurrently with A being CH₂;
R_{27A} is H concurrently with R_{27B} being CH₃, or R_{27A} is CH₃ concurrently with R_{27B} being H;
R₃ comprises a glycosyl group linked through the oxygen atom to the steroidal sapogenin at C-3; or a pharmaceutically acceptable salt, or derivative thereof. 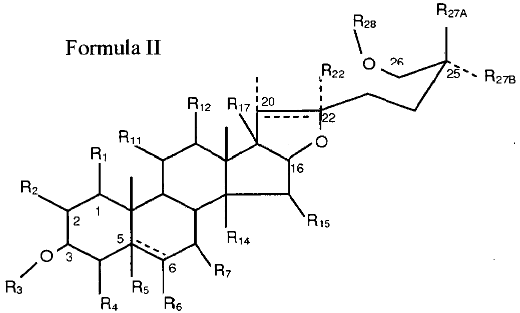 wherein
R₁, R₂, R₄, R₆, R₇, R₁₁, R₁₂, R₁₄, R₁₅ and R₁₇ are independently H, OH, =O, pharmacologically acceptable ester groups or pharmacologically acceptable ether groups;
R₅ is H when C-5,C-6 is a single bond, and nothing when C-5,C-6 is a double bond;
R₂₂ is either a hydroxyl or an alkoxyl group when C-20, C-22 is a single bond, or nothing when C-20, C-22 is a double bond;
R_{27A} is H concurrently with R_{27B} being CH₃, or R_{27A} is CH₃ concurrently with R_{27B} being H;
R₂₈ is H or a saccharide; or a pharmaceutically acceptable salt, or derivative thereof;
R₃ comprises a glycosyl group linked through the oxygen atom to the steroidal sapogenin at C-3; or a pharmaceutically acceptable salt, or derivative thereof.

Examples of steroid sapogenins include spirostanol aglycones such as diosgenin, yamogenin (neodiosgenin), yuccagenin, sarsasapogenin, tigogenin, smilagenin, hecogenin, gitogenin, convallamarogenin, neoruscogenin, and solagenin; and furostanol aglycones such as protodiosgenin, pseudoprotodiosgenin, methyl protodiosgenin, protoyamogenin, and methyl protoyamogenin.

Examples of chacotrioside-steroid saponins include "dioscin" which consists of the sapogenin "diosgenin" linked through the C-3 position to chacotriose, diosgenin linked through the C-3 position to another chacotrioside, tigogenin linked through the C-3 position to a chacotrioside, sarsasapogenin linked through the C-3 position to a chacotrioside, smilagenin linked through the C-3 position to a chacotrioside, yuccagenin linked through the C-3 position to a chacotrioside, and yamogenin linked through the C-3 position to a chacotrioside.

Examples of solatrioside steroid saponins include "gracillin", which is diosgenin linked through the C-3 position to the solatrioside (Rha 2, [Glc 3], Glc); deltonin (diosgenin linked through the C-3 position to the solatrioside Rha 2, [Glc 4], Glc); diosgenin linked through the C-3 position to solatriose (Rha 2, [Glc 3], Gal) [in this context, diosgenin linked to (Rha 2, [Glc 3], Gal) is termed 'diosgenin solatriose']; diosgenin linked through the C-3 position to another solatrioside; tigogenin linked through the C-3 position to a solatrioside; sarsasapogenin linked through the C-3 position to a solatrioside; smilagenin linked through the C-3 position to a solatrioside; yuccagenin linked through the C-3 position to a solatrioside, and yamogenin linked through the C-3 position to a solatrioside.

Simple monosaccharide steroid saponins are widespread in the plant kingdom. The monosaccharide is generally linked to the aglycone through the C-3 position and examples include "trillin," which is diosgenin linked through the C-3 position to glucose. Other sapogenins linked to glucose through the C-3 position include sarsasapogenin, rhodeasapogenin and yamogenin. Some sapogenins are linked through the C-3 position to another monosaccharide such as arabinose eg, yonogenin and convallagenin or linked through the C-3 position to galactose and so forth.

Examples of disaccharide steroid saponins include sarsasapogenin linked through the C-3 position to for example (Xyl 2, Gal 3); (Glc 2, Glc 3); (Glc 3, Glc 3); smilagenin linked through the C-3 position to (Glc 2, Glc 3); (Glc 2, Gal 3); samogenin, tigogenin, gitogenin, alliogenin, ruscogenin, pennogenin, cepagenin and diosgenin linked through the C-3 position to (Rha 2, Glc 3)

The diosgenin glycosides from *Dioscorea* species are of great commercial interest as starting materials for steroid hormones. Glycosides of diosgenin and its C-25 isomer yamogenin are among the most frequently documented spirostanol saponins.

Examples of naturally occurring steroid spirostanol sapogenins with a C-5,C-6 double bond in the B-ring are listed in Table 1:

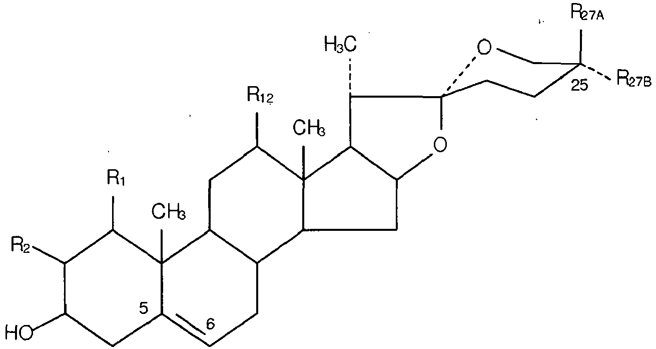

**Table 1**

| | R₁ | R₂ | R₁₂ | R_{27A} | R_{27B} |
|---|---|---|---|---|---|
| Diosgenin | H | H | H | H | CH₃ |
| Yamogenin | H | H | H | CH₃ | H |
| Yuccagenin | H | OH | H | H | CH₃ |
| Gentrogenin | H | H | =O | H | CH₃ |
| Ruscogenin | OH | H | H | H | CH₃ |

Examples of naturally occurring steroid spirostanol sapogenins with a C-5, C-6 single bond in the B-ring are listed in Table 2:

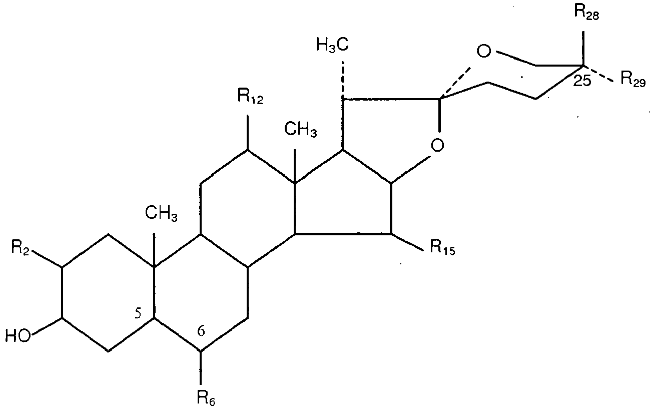

**Table 2**

| | R₂ | H₅ | R₆ | R₁₂ | R₁₅ | R₂₈ | R₂₉ |
|---|---|---|---|---|---|---|---|
| Smilagenin | H | β | H | H | H | H | CH₃ |
| Tigogenin | H | α | H | H | H | H | CH₃ |
| Sarsasapogenin | H | β | H | H | H | CH₃ | H |
| Gitogenin | OH | β | H | H | H | H | CH₃ |
| Hecogenin | H | α | H | =O | H | H | CH₃ |
| Chlorogenin | H | α | OH(α) | H | H | H | CH₃ |
| Digitogenin | OH(α) | α | H | H | OH(β) | H | CH₃ |
| Digalogenin | H | α | H | H | OH(β) | H | CH₃ |

Examples of naturally occurring steroid furostanol sapogenins of the protospirostane-type with a C-5,C-6 double bond in the B-ring and a C-20,C-22 single bond in the E-ring, are listed in Table 3:

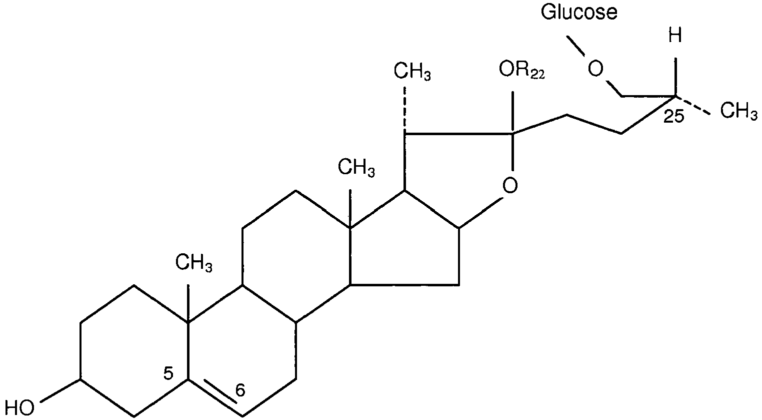

**Table 3**

| | R₂₂ |
|---|---|
| Protodiosgenin | H |
| Methyl protodiosgenin | CH₃ |

An example of a naturally occurring steroid furostanol sapogenin of the protospirostane-type with a C-5, C-6 single bond in the B-ring and a C-20, C-22 single bond in the E-ring, is prototigogenin.

An example of a naturally occurring steroid furostanol sapogenin of the pseudospirostane-type with a C-5,C-6 double bond in the B-ring and a C-20,C-22 double bond in the E-ring is pseudodiosgenin.

An example of a naturally occurring steroid furostanol sapogenin of the pseudoprotospirostane-type with a C-5,C-6 double bond in the B-ring and a C-20,C-22 double bond in the E-ring is pseudoprotodiosgenin.

In one embodiment, the steroid saponin is dioscin, where dioscin is the sapogenin diosgenin linked through the C-3 position to chacotriose (Rha 2, [Rha 4], Glc).

In another embodiment, the steroid saponin is deltonin where deltonin is the sapogenin diosgenin linked through the C-3 position to Rha 2, [Glc 4], Glc.

In one specific embodiment, the steroid saponin is selected from the group consisting of deltonin (diosgenin Rha2, [Glc4], Glc), dioscin (diosgenin Rha2, [Rha4], Glc), and prosapogenin A (diosgenin Rha2, Glc).

In the case of deltonin and prosapogenin A, any one of these steroid saponins may be prepared in a pharmaceutical composition.

Accordingly, such steroid saponins may be used in the preparation of a medicament.

As discussed previously herein, the steroid saponin in the various embodiments of the present invention may be obtained from natural sources, manufactured from synthesis processes, or as partial synthesis or modification applied to naturally occurring compounds or intermediates.

The extraction, isolation and identification of the steroid saponin in the various embodiments of the present invention may be achieved by methods known in the art.

For example, some steroid saponins are produced from plant sources. Other sources of steroid saponins may be readily obtained from the literature, for example as described in Hostettmann K and Marston A (2005). Chemistry & pharmacology of natural products: Saponins. Cambridge University Press, chapters 1-3 and 6. Common names of steroid saponins have been used in accordance with the above text and the Dictionary of Natural Products, Chapman and Hall, CRC, (2004).

The amount of the steroid saponin administered to the biological system in the various embodiments of the present invention is not particularly limited, and generally will be in the range such that the target will be exposed to a concentration from 0.1 µM to 20 µM of the steroid saponin.

The steroid saponin may be delivered in a form and at a concentration suitable to allow the agent to reach the desired site of action and have the desired effect, such as inhibiting angiogenesis.

The administration of the steroid saponin may be within any time suitable to produce the desired effect. In a human or animal subject, the steroid saponin may be administered for example orally, parenterally, topically or by any other suitable means, and therefore transit time of the agent must be taken into account.

For example, the administration of the steroid saponin to the subject in the various embodiments of the present invention may be at one or more of prior to the start of angiogenesis, and/or concurrently with angiogenesis occurring. For example, in the case of inhibiting angiogenesis associated with a solid cancer, the administration of the steroid saponin may be before and/or during the growth of a tumour (primary and/or secondary tumours), and/or before or after resection of a tumour (primary and/or secondary tumours).

The steroid saponin in the various embodiments of the present invention may be administered to the subject in a suitable form.

In one embodiment, the steroid saponin is in the form of a composition suitable for use to inhibit angiogenesis.

Accordingly, in another embodiment the present invention provides a composition when used to inhibit angiogenesis in a biological system, the composition including an effective amount of a steroid saponin.

The effective amount of the steroid saponin to be administered to the biological system (eg a subject) is not particularly limited, so long as it is within such an amount and in such a form that generally exhibits a useful or therapeutic effect. The term "therapeutically effective amount" is the quantity which, when administered to a subject in need of treatment, improves the prognosis and/or state of the subject. The amount to be administered to a subject will depend on the particular characteristics of the angiogenesis to be inhibited, the mode of administration, and the characteristics of the subject, such as general health, other diseases, age, sex, genotype, and body weight. A person skilled in the art will be able to determine appropriate dosages depending on these and other factors.

Accordingly, in another embodiment the present invention provides use of a steroid saponin in the preparation of a medicament for inhibiting angiogenesis in a biological system.

As discussed previously herein, administration and delivery of the steroid saponin may be, for example, by the intravenous, intraperitoneal, subcutaneous, intramuscular, oral, or topical route, or by direct injection into the desired site of action. The mode and route of administration in most cases will depend on the type of disease or condition being treated.

The dosage form, frequency and amount of dose will depend on the mode and route of administration. Typically an injectable composition will be administered in an amount of between 5 mg/m² and 500 mg/m², generally between 10 mg/m² and 200 mg/m². Typically an orally administered composition will be administered in an amount of between 5 mg and 5 g, preferably between 50 mg and 1 g.

For example, an effective amount of the steroid saponin typically ranges between about 0.1 mg/kg body weight per day and about 1000 mg/kg body weight per day, and in one form between 1 mg/kg body weight per day and 100 mg/kg body weight per day.

As described above, the administration of the compositions of steroid saponin may also include the use of one or more pharmaceutically acceptable additives, including pharmaceutically acceptable salts, amino acids, polypeptides, polymers, solvents, buffers, excipients, preservatives and bulking agents, taking into consideration the particular physical, microbiological and chemical characteristics of the steroid saponin to be administered.

For example, the steroid saponin can be prepared into a variety of pharmaceutical acceptable compositions in the form of, e.g., an aqueous solution, an oily preparation, a fatty emulsion, an emulsion, a lyophilised powder for reconstitution, etc. and can be administered as a sterile and pyrogen free intramuscular or subcutaneous injection or as injection to an organ, or as an embedded preparation or as a transmucosal preparation through nasal cavity, rectum, uterus, vagina, lung, etc. The composition may be administered in the form of oral preparations (for example solid preparations such as tablets, caplets, capsules, granules or powders; liquid preparations such as syrup, emulsions, dispersions or suspensions).

Compositions containing the steroid saponin may also contain one or more pharmaceutically acceptable preservative, buffering agent, diluent, stabiliser, chelating agent, viscosity-enhancing agent, dispersing agent, pH controller, solubility-modifying agent or isotonic agent. These excipients are well known to those skilled in the art.

Examples of suitable preservatives are benzoic acid esters of para-hydroxybenzoic acid, phenols, phenylethyl alcohol or benzyl alcohol. Examples of suitable buffers are sodium phosphate salts, citric acid, tartaric acid and the like. Examples of suitable stabilisers are antioxidants such as alpha-tocopherol acetate, alpha-thioglycerin, sodium metabisulphite, ascorbic acid, acetylcysteine, 8-hydroxyquinoline, and chelating agents such as disodium edetate. Examples of suitable viscosity enhancing agents, suspending, solubilizing or dispersing agents are substituted cellulose ethers, substituted cellulose esters, polyvinyl alcohol, polyvinylpyrrolidone, polyethylene glycols, carbomer, polyoxypropylene glycols, sorbitan monooleate, sorbitan sesquioleate, polyoxyethylene hydrogenated castor oil 60.

Examples of suitable pH controllers include hydrochloric acid, sodium hydroxide, buffers and the like. Examples of suitable isotonic agents are glucose, D-sorbitol or D-mannitol, sodium chloride.

The administration of the steroid saponin may also be in the form of a composition containing a pharmaceutically acceptable carrier, diluent, excipient, suspending agent, lubricating agent, adjuvant, vehicle, delivery system, emulsifier, disintegrant, absorbent, preservative, surfactant, colorant, glidant, anti-adherent, binder, flavorant or sweetener, taking into account the physical, chemical and microbiological properties of the steroid saponin being administered.

For these purposes, the composition may be administered orally, parenterally, by inhalation spray, adsorption, absorption, topically, rectally, nasally, bucally, vaginally, intraventricularly, via an implanted reservoir in dosage formulations containing conventional non-toxic pharmaceutically-acceptable carriers, or by any other convenient dosage form. The term parenteral as used herein includes subcutaneous, intravenous, intramuscular, intraperitoneal, intrathecal, intraventricular, intrasternal, and intracranial injection or infusion techniques.

When administered parenterally, the composition will normally be in a unit dosage, sterile, pyrogen free injectable form (solution, suspension or emulsion, which may have been reconstituted prior to use) which is usually isotonic with the blood of the recipient with a pharmaceutically acceptable carrier. Examples of such sterile injectable forms are sterile injectable aqueous or oleaginous suspensions. These suspensions may be formulated according to techniques known in the art using suitable vehicles, dispersing or wetting agents, complexing agents, polymers, solubility aids and suspending agents. The sterile injectable forms may also be sterile injectable solutions or suspensions in non-toxic parenterally acceptable diluents or solvents, for example, as solutions in 1,3-butanediol. Among the pharmaceutically acceptable vehicles and solvents that may be employed are water, ethanol, glycerol, saline, dimethylsuphoxide, N-methylpyrrolidone, dimethylacetamide, Ringer's solution, dextrose solution, isotonic sodium chloride solution, and Hanks' solution. In addition, sterile, fixed oils are conventionally employed as solvents or suspending mediums. For this purpose, any bland fixed oil may be employed including synthetic mono- or di-glycerides, corn, cottonseed, peanut, and sesame oil. Fatty acids such as ethyl oleate, isopropyl myristate, and oleic acid and its glyceride derivatives, including olive oil and castor oil, especially in their polyoxyethylated versions, are useful in the preparation of injectables. These oil solutions or suspensions may also contain long-chain alcohol diluents or dispersants.

The carrier may contain additives, such as substances that enhance solubility, isotonicity, and chemical stability, for example anti-oxidants, buffers and preservatives.

In addition, the composition containing the steroid saponin may be in a form to be reconstituted prior to administration. Examples include lyophilization, spray drying and the like to produce a suitable solid form for reconstitution with a pharmaceutically acceptable solvent prior to administration.

Compositions may include one or more buffer, bulking agent, isotonic agent and cryoprotectant and lyoprotectant. Examples of excipients include, phosphate salts, citric acid, non-reducing sugars such as sucrose or trehalose, polyhydroxy alcohols, amino acids, methylamines, and lyotropic salts are preferred to the reducing sugars such as maltose or lactose.

When administered orally, the steroid saponin will usually be formulated into unit dosage forms such as tablets, caplets, cachets, powder, granules, beads, chewable lozenges, capsules, liquids, aqueous suspensions or solutions, or similar dosage forms, using conventional equipment and techniques known in the art. Such formulations typically include a solid, semisolid, or liquid carrier. Exemplary carriers include excipients such as lactose, dextrose, sucrose, sorbitol, mannitol, starches, gum acacia, calcium phosphate, mineral oil, cocoa butter, oil of theobroma, alginates, tragacanth, gelatin, syrup, substituted cellulose ethers, polyoxyethylene sorbitan monolaurate, methyl hydroxybenzoate, propyl hydroxybenzoate, talc, magnesium stearate, and the like.

A tablet may be made by compressing or moulding the steroid saponin optionally with one or more accessory ingredients. Compressed tablets may be prepared by compressing, in a suitable machine, the active ingredient in a free-flowing form such as a powder or granules, optionally mixed with a binder, lubricant, inert diluent, surface active, or dispersing agent. Moulded tablets may be made by moulding in a suitable machine, a mixture of the powdered active ingredient and a suitable carrier moistened with an inert liquid diluent.

The administration of the steroid saponin may also utilize controlled release technology.

For topical administration, the composition may be in the form of a solution, spray, lotion, cream (for example a non-ionic cream), gel, paste or ointment. Alternatively, the composition may be delivered via a liposome, nanosome, ribosome, or nutri-diffuser vehicle.

As discussed previously herein, the effective amount of the steroid saponin to be administered is not particularly limited, so long as it is within such an amount and in such a form that generally exhibits a pharmacologically useful or therapeutic effect.

The administration of the steroid saponin may further include the administration of an anti-angiogenic agent, including anti-VEGF antibodies, including humanized and chimeric antibodies, anti-VEGF aptamers and antisense oligonucleotides, angiostatin, endostatin, interferons, interleukin 1, interleukin 12, retinoic acid, and tissue inhibitors of metalloproteinase-2 and -9.

The administration route, dose and treatment regimnes of anti-angiogenic agents may be determined by a person skilled in the art. Anti-angiogenic agents and their use are generally as described in "Biotherapy: A comprehensive Overview" (2001) Second Edition, ed. By Paula Trahan Rieger, Jones and Bartlett Publishers International.

The inhibition of angiogenesis in the biological system may be determined by a suitable method known in the art, such as delayed appearance of neovascular structures, slowed development of neovascular structures, decreased occurrence of neovascular structures, slowed or decreased severity of angiogenesis-dependent disease effects, arrested angiogenic growth, or regression of previous angiogenic growth.

Determination of the ability of the steroid saponin to inhibit angiogenesis may be by any suitable assay of measuring angiogenesis that is well known in the art. For example, a mouse aortic explant model may be used.

Alternatively, chicken chorioallantoic membrane (CAM) assay or a corneal neovascularization model may be performed. The ability of steroid saponin to inhibit angiogenesis may be determined by the extent of inhibition of angiogenesis in the chicken embryo or the extent of inhibition of angiogenesis in a corneal neovascularization model.

For example, the ability of a steroid saponin to inhibit angiogenesis in a chicken chorioallantoic membrane assay may be tested by contacting the chorioallantoic membrane with the steroid saponin applied to a methylcellulose disc. For the corneal neovascularization model, the steroid saponin may be applied as a topical composition containing the steroid saponin to the cornea, the cornea being scratched and inoculated with an agent to induce neovascularisation.

Another method to study angiogenesis is the subcutaneous implantation of various artificial sponges (i.e. polyvinyl alcohol, gelatin) in animals. The steroid saponin to be evaluated may be injected directly into the sponges, which are placed in the animal. Neovascularization of the sponges is assessed either histologically, morphometrically (vascular density), biochemically (haemoglobin content) or by measuring the blood flow rate in the vasculature of the sponge using a radioactive tracer.

Numerous animal tumour models have also been developed to test the anti-angiogenic activity of test compounds. In many cases, tumour cells are engrafted subcutaneously and tumour size is determined at regular time intervals. Frequently used tumour cells include C6 rat glioma, B16BL6 melanoma, LLC, and Walker 256 carcinoma.

It is also contemplated that the steroid saponin of the present invention will be suitable for use to reduce the amount of an anti-angiogenic agent administered to a biological system.

Accordingly, in another embodiment the present invention also provides a method of reducing the amount of an anti-angiogenic agent administered to a biological system to achieve a desired level of inhibition of angiogenesis, the method including the step of administering to the biological system an effective amount of a steroid saponin.

The effective amount of the steroid saponin to be administered is not particularly limited, so long as it is within such an amount that generally exhibits a pharmacologically useful effect to reduce the amount of agent necessary to achieve a desired level of inhibition of angiogenesis in the biological system.

The administration of the steroid saponin may be within any time suitable to produce the desired effect of reducing the amount of an agent administered to a biological system necessary to achieve a desired level of inhibition of angiogenesis in the biological system. As discussed previously herein, in a human or animal subject the steroid saponin may be administered for example orally, parenterally, topically or by any other suitable means, and therefore transit time of the drug must be taken into account.

As described previously herein, examples of anti-angiogenic agents include anti-VEGF antibodies, including humanized and chimeric antibodies, anti-VEGF aptamers and antisense oligonucleotides, angiostatin, endostatin, interferons, interleukin 1, interleukin 12, retinoic acid, and tissue inhibitors of metal loproteinase-2 and -9.

In this regard, the amount of the anti-angiogenic agent necessary to achieve a desired level of inhibition of angiogenesis will be empirically determined by a method known in the art, and as such will depend upon the desired level of angiogenesis to be inhibited, the age and body weight of the subject, and the frequency of administration.

The administration of the anti-angiogenic agent will be in a suitable form and within a suitable time to produce the desired effect of inhibiting angiogenesis to the desired level. Methods for formulating and administering anti-angiogenic agents are known in the art.

The administration of the steroid saponin may occur at the same time and in the same manner as the administration of the anti-angiogenic agent. Alternatively, the administration of the steroid saponin may be separate to the administration of the anti-angiogenic agent, and occur at a pharmacologically appropriate time before or after administration of the agent.

The present invention also provides a combination product for separate or co-administration of a steroid saponin and an anti-angiogenic agent to a subject to inhibit angiogenesis. In this case, the combination product includes the steroid saponin for separate administration to the subject in a suitable form, or alternatively, for co-administration to the subject in a suitable form.

Accordingly, in another embodiment the present invention provides a combination product including a steroid saponin and an anti-angiogenic agent, the steroid saponin and the anti-angiogenic agent provided in a form for co-administration to a subject or in a form for separate administration to a subject.

The components of the combination product may be packaged separately or together in suitably sterilized containers such as ampoules, bottles, or vials, either in multi-dose or in unit dosage forms. The containers are typically hermetically sealed. Methods are known in the art for the packaging of the components.

As discussed previously herein, co-administration can be sequential or simultaneous and generally means that the agents are present in the subject during a specified time interval. Typically, if a second agent is administered within the half-life of the first agent, the two agents are considered co-administered.

The present invention may be used to inhibit endothelial cell proliferation and/or migration.

Accordingly, in another embodiment the present invention provides a method of inhibiting endothelial cell proliferation and/or migration in a biological system, the method including the step of administering to the biological system an effective amount of a steroid saponin.

In this regard, it will be appreciated that the biological system is any biological system in which endothelial cell proliferation and/or migration is occurring or in which angiogenesis may occur.

In one embodiment, the biological system is a human or animal subject.

In a further embodiment, the biological system is a human or animal subject in which the endothelial cell proliferation and/or migration is associated with a disease or condition that is due to undesired or uncontrolled endothelial cell proliferation and/or migration.

For example, the biological system may be a human or animal subject suffering from, or susceptible to, endothelial cell proliferation and/or migration associated with the formation or expansion of angiofibroma, corneal neovascularisation, retinal/choroidal neovascularization, diabetic retinopathy, age-related macular degeneration, arteriovenous malformations, arthritis, rheumatoid arthritis, osteoarthritis, psoriatic arthritis, lupus, connective tissue disorders, Osler-Weber syndrome, atherosclerotic plaques, psoriasis, pyogenic granuloma, retrolental fibroplasias, scleroderma, granulations, hemangioma; trachoma, haemophilic joints, vascular adhesions, hypertrophic scars, diseases or conditions associated with acute or chronic inflammation, diseases or conditions associated with chronic inflammation of the lung including asthma, sarcoidosis, inflammatory bowel diseases, Crohn's disease or ulcerative colitis.

The amount of the steroid saponin administered to the biological system is generally will be in the range such that the target endothelial cells will be exposed to a concentration from 0.1 µM to 20 µM of the steroid saponin.

The steroid saponin may be delivered in a form and at a concentration suitable to allow the agents to reach the desired site of action and have the effect of inhibiting endothelial cell proliferation and/or migration.

The administration of the steroid saponin may be within any time suitable to produce the desired effect of inhibiting endothelial cell proliferation and/or migration. In a human or animal subject, the steroid saponin may be administered for example orally, parenterally, topically or by any other suitable means, and therefore transit time of the agent must be taken into account.

For example, the administration of the steroid saponin to the subject in the various embodiments of the present invention may be at one or more of prior to the start of endothelial cell proliferation and/or migration, and/or concurrently with endothelial cell proliferation and/or migration occurring.

As discussed previously herein, the steroid saponin may be formulated and administered to the subject in a suitable form and in a suitable manner.

In one embodiment, the steroid saponin is in the form of a composition suitable for use to inhibit angiogenesis.

Accordingly, in another embodiment the present invention provides a composition when used to inhibit endothelial cell proliferation and/or migration in a biological system, the composition including an effective amount of a steroid saponin.

The effective amount of the steroid saponin to be administered to the subject is not particularly limited, so long as it is within such an amount and in such a form that generally exhibits a useful or therapeutic effect. The term "therapeutically effective amount" is the quantity which, when administered to a subject in need of treatment, improves the prognosis and/or state of the subject. The amount to be administered to a subject will depend on the particular characteristics of the endothelial cell proliferation and/or migration to be inhibited, the mode of administration, and the characteristics of the subject, such as general health, other diseases, age, sex, genotype, and body weight. A person skilled in the art will be able to determine appropriate dosages depending on these and other factors.

Accordingly, in another embodiment the present invention provides use of a steroid saponin in the preparation of a medicament for inhibiting endothelial cell proliferation and/or migration in a biological system.

As discussed previously herein, administration and delivery of the steroid saponin may be, for example, by the intravenous, intraperitoneal, subcutaneous, intramuscular, oral, or topical route, or by direct injection into the desired site of action. The mode and route of administration in most cases will depend on the type of disease or condition being treated, as discussed previously herein.

The dosage form, frequency and amount of dose will depend on the mode and route of administration. Typically an injectable composition will be administered in an amount of between 5 mg/m² and 500 mg/m², preferably between 10 mg/m² and 200 mg/m² Typically an orally administered composition will be administered in an amount of between 5 mg and 5 g, preferably between 50 mg and 1 g.

For example, an effective amount of the steroid saponin typically ranges between about 0.1 mg/kg body weight per day and about 1000 mg/kg body weight per day, and in one form between 1 mg/kg body weight per day and 100 mg/kg body weight per day.

As described previously herein, the administration of the compositions of the steroid saponin may also include the use of one or more pharmaceutically acceptable additives, including pharmaceutically acceptable salts, amino acids, polypeptides, polymers, solvents, buffers, excipients, preservatives and bulking agents, taking into consideration the particular physical, microbiological and chemical characteristics of the steroid saponin to be administered.

As described previously herein, compositions containing the steroid saponin may also contain one or more pharmaceutically acceptable preservative, buffering agent, diluent, stabiliser, chelating agent, viscosity-enhancing agent, dispersing agent, pH controller, or isotonic agent. These excipients are well known to those skilled in the art.

The administration of the steroid saponin may further include the administration of an anti-angiogenic agent, including anti-VEGF antibodies, including humanized and chimeric antibodies, anti-VEGF aptamers and antisense oligonucleotides, angiostatin, endostatin, interferons, interleukin 1, interleukin 12, retinoic acid, and tissue inhibitors of metalloproteinase-2 and -9.

The inhibition of the proliferation and/or migration of endothelial cells in the biological system may be determined by a suitable method known in the art.

For example, in the case of cell proliferation, methods such as cell counting, 3[H] thymidine incorporation, immuno-histochemical staining for cell proliferation, delayed appearance of neovascular structures, slowed development of neovascular structures, decreased occurrence of neovascular structures, slowed or decreased severity of angiogenesis-dependent disease effects, arrested angiogenic growth, or regression of previous angiogenic growth.

The determination of the ability of a steroid saponin to inhibit proliferation of endothelial cells may be by a suitable assay known in the art in which cells are treated and endothelial cell proliferation measured. For example, human umbilical vascular endothelial cells may be cultured *in vitro* in the appropriate medium and endothelial cell proliferation may be measured, for example, by tritiated thymidine uptake. The ability of an agent to inhibit proliferation in such an assay may then be tested by contacting the endothelial cells with the agent and determining the extent of inhibition of proliferation that occurs at any particular concentration.

In the case of endothelial cell migration, a suitable assay is the BD BioCoat™ Angiogenesis System: Endothelial Cell Migration.

It is also contemplated that the steroid saponin of the present invention will be suitable for use to reduce the amount of an anti-angiogenic agent administered to a biological system.

Accordingly, in another embodiment the present invention provides a method of reducing the amount of an anti-angiogenic agent administered to a biological system to achieve a desired level of inhibition of endothelial cell proliferation and/or migration, the method including the step of administering to the biological system an effective amount of a steroid saponin.

The effective amount of the steroid saponin to be administered is not particularly limited, so long as it is within such an amount that generally exhibits a pharmacologically useful effect to reduce the amount of agent necessary to achieve a desired level of inhibition of endothelial cell proliferation and/or migration in the biological system.

The administration of the steroid saponin may be within any time suitable to produce the desired effect of reducing the amount of an agent administered to a biological system necessary to achieve a desired level of inhibition of endothelial cell proliferation and/or migration in the biological system. As discussed previously herein, in a human or animal subject the steroid saponin may be administered for example orally, parenterally, topically or by any other suitable means, and therefore transit time of the drug must be taken into account.

As described previously herein, examples of anti-angiogenic agents include anti-VEGF antibodies, including humanized and chimeric antibodies, anti-VEGF aptamers and antisense oligonucleotides, angiostatin, endostatin, interferons, interleukin 1, interleukin 12, retinoic acid, and tissue inhibitors of metalloproteinase-2 and -9.

In this regard, the amount of the anti-angiogenic agent necessary to achieve a desired level of inhibition of endothelial cell proliferation and/or migration will be empirically determined by a method known in the art, and as such will depend upon the desired level to be inhibited, the age and body weight of the subject, and the frequency of administration.

The administration of the anti-angiogenic agent will be in a suitable form and within a suitable time to produce the desired effect of inhibiting angiogenesis to the desired level. Methods for formulating and administering anti-angiogenic agents are known in the art.

The administration of the steroid saponin may occur at the same time and in the same manner as the administration of the anti-angiogenic agent. Alternatively, the administration of the steroid saponin may be separate to the administration of the anti-angiogenic agent, and occur at a pharmacologically appropriate time before or after administration of the agent.

The present invention also provides a combination product for separate or co-administration of a steroid saponin and an anti-angiogenic agent to a subject to inhibit endothelial cell proliferation and/or migration. In this case, the combination product includes the steroid saponin for separate administration to the subject in a suitable form, or alternatively, for co-administration to the subject in a suitable form.

Accordingly, in another embodiment the present invention provides a combination product including a steroid saponin and an anti-angiogenic agent, the steroid saponin and the anti-angiogenic agent provided in a form for co-administration to a subject or in a form for separate administration to a subject.

The components of the combination product may be packaged separately or together in suitably sterilized containers such as ampoules, bottles, or vials, either in multi-dose or in unit dosage forms. The containers are typically hermetically sealed. Methods are known in the art for the packaging of the components.

As discussed previously herein, co-administration can be sequential or simultaneous and generally means that the agents are present in the subject during a specified time interval. Typically, if a second agent is administered within the half-life of the first agent, the two agents are considered co-administered.

Methods for the preparation of pharmaceutical compositions are known in the art, for example as described in Remington's Pharmaceutical Sciences, 18th ed., 1990, Mack Publishing Co., Easton, Pa.; U.S. Pharmacopeia: National Formulary, 1984, Mack Publishing Company, Easton, Pa.; and M.E. Aulton, Pharmaceutics, The Science of Dosage Form Design, 2nd ed., Churchill Livingstone, Edinburgh, 2002.

Therapeutic delivery of biolomolecules is generally as described in Bladon, C. (2002) "Pharmaceutical Chemistry: Therapeutic Aspects of Biomolecules" John Wiley & Sons Ltd.

### Description of Specific Embodiments

Reference will now be made to experiments that embody the above general principles of the present invention.

### Example 1

### Materials

Diosgenin, dioscin (diosgenin Rha2, [Rha4], Glc), deltonin (diosgenin Rha2, [Glc4], Glc) and trillin (diosgenin-Glc) were obtained commercially from Ningbo Hanpharm Biotech Co Ltd, gracillin from ChromaDex, and trillin from Aktin Chemicals. Prosapogenin A: diosgenin Rha2, Glc was synthesised in accordance with the method described by Li et al Carbohydr. Res., (2001) 331, 1-7. Dioscin and prosapogenin A were also isolated from *Paris polyphylla.* Sorafenib was obtained commercially.

### Example 2

### Assessment of the effects of deltonin on vessel growth in aortic explants

Three male C57BL6/J mice (9 weeks old) were sacrificed by CO₂ asphyxiation and the blood harvested by heart puncture using a 29 gauge needle. The aorta was then dissected (from the aortic arch to the pleural/peritoneal interface) and placed in ice-cold DMEM supplemented with 10 mM Hepes and penicillin/streptomycin.

The fibrous and adipose tissues surrounding the aorta were removed by microscopic dissection and the aorta flushed of blood using ice cold DMEM. The aorta was then longitudinally bisected and cut into approximately 1 mm squares. Individual pieces of aorta were embedded in a drop (20 µL) of collagen solution in the bottom of a well of a 24 well tissue culture plate. The plates were then placed at 37°C/5% CO₂ for 10 minutes to polymerise the collagen into a gel. Media (0.3 mL) alone, containing DMSO or deltonin in DMSO was then added to each well to form a moat around the collagen gel. Only the central 8 wells of each 24 well plate were used for explant cultures to avoid differential gaseous exchange and evaporation rates between explant sample wells. The outer wells were filled with PBS.

Nine groups of 8 replicates each were tested:
1. Media alone (EBM-2 supplemented with 10 mM Hepes and 2 % normal mouse serum (I).
2. Media plus 1 µL/mL DMSO (diluent control for deltonin) (H).
3. Media plus 1 µL/mL DMSO, 10 nM deltonin (G).
4. Media plus 1 µL/mL DMSO, 30 nM deltonin (F).
5. Media plus 1 µL/mL DMSO, 100 nM deltonin (E).
6. Media plus 1 µL/mL DMSO, 300 nM deltonin (D).
7. Media plus 1 µL/mL DMSO, 1 µM deltonin (C).
8. Media plus 1 µL/mL DMSO, 3 µM deltonin (B).
9. Media plus 1 µL/mL DMSO, 10 µM deltonin (A).

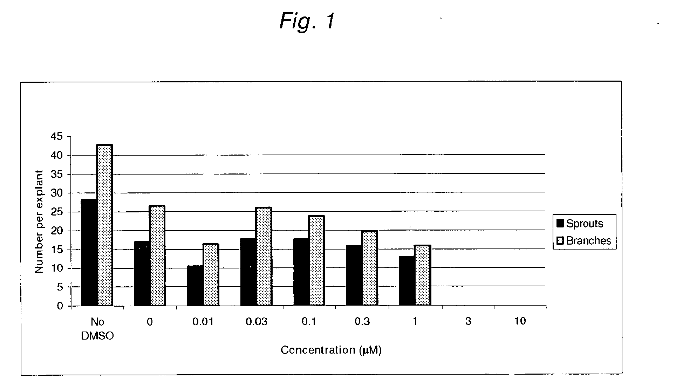

The explants were then cultured at 37°C/5% CO₂ in a humid incubator for 6 days. Explants were fixed at room temperature for 20 minutes by adding an equal volume (0.3 mL) of 4% paraformaldehyde in PBS to each well. After washing three times with Hepes-buffered saline (HBS, 150 mM NaCl, 10 mM Hepes) the explants were stained with 10 µg/ml FITC-BS-1 lectin conjugate (BS-1 lectin specifically binds mouse endothelial cells) in HBS overnight at 4°C. The following day the explants were washed three times with HBS. Vessel outgrowths from explants were visualised by fluorescence microscopy and photographs taken for data analysis. Multiple multidepth focus fluorescent images were photographed for each explant and the photographs processed using deconvolution software so that total vessel outgrowth for each explant could be determined. Total cellular growth associated with each explant was also visualised by light microscopy using a dissecting microscope and documented by photography. For ease of visualisation and analysis a composite inverted black and white image was prepared from the multiple fluorescent images of the vessel outgrowths for each explant. The number of vessel sprouts and the number of vessel branches for each explant was then counted with the person counting being blinded to the identity of each sample. The results are summarised in Table 4, and shown graphically in Figure 1.

**Table 4**

| Concentration of Deltonin (µM) | Sprouts | | Branches | |
|---|---|---|---|---|
| | Mean | SD | Mean | SD |
| No DMSO | 28 | 10 | 43 | 17 |
| 0 | 17 | 5 | 26 | 9 |
| 0.01 | 11 | 7 | 16 | 10 |
| 0.03 | 18 | 6 | 26 | 9 |
| 0.1 | 18 | 6 | 24 | 9 |
| 0.3 | 16 | 4 | 20 | 5 |
| 1 | 13 | 5 | 16 | 7 |
| 3 | 0 | 0 | 0 | 0 |
| 10 | 0 | 0 | 0 | 0 |

| | | | | |
|---|---|---|---|---|
| Note: vessel sprouts and branching in explants cultured with 0.01 µM Deltonin are not statistically different from DMSO treated control explant cultures despite an apparent reduction on the graph. | | | | |

There was a significant inhibitory effect alone of DMSO, the vehicle for deltonin, on the angiogenic response as observed by significant reductions in vessel sprouting and branching in aortic explants treated with DMSO in comparison to aortic explants cultured in the absence of DMSO.

Nevertheless, high concentrations of deltonin significantly inhibited vessel sprouts (at 10 and 3 µM) and vessel branches (at 10, 3 and 1 µM) from aortic explants in comparison to DMSO treated control explant cultures.

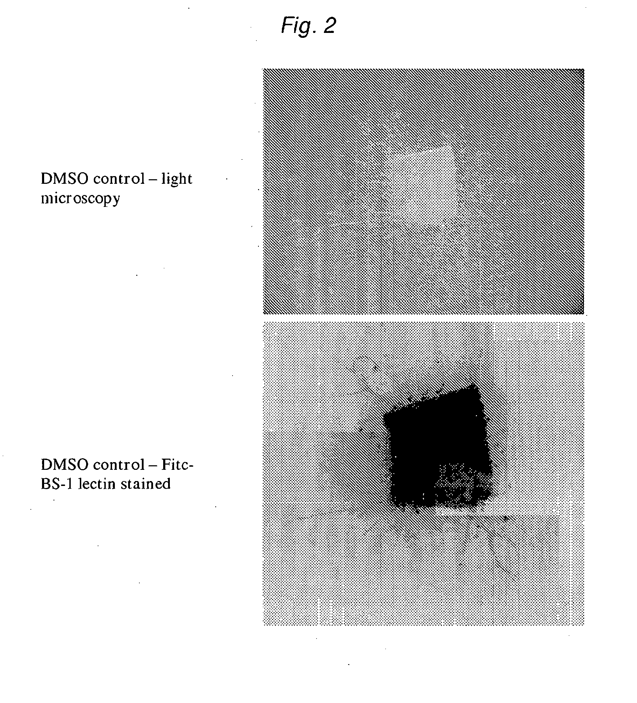

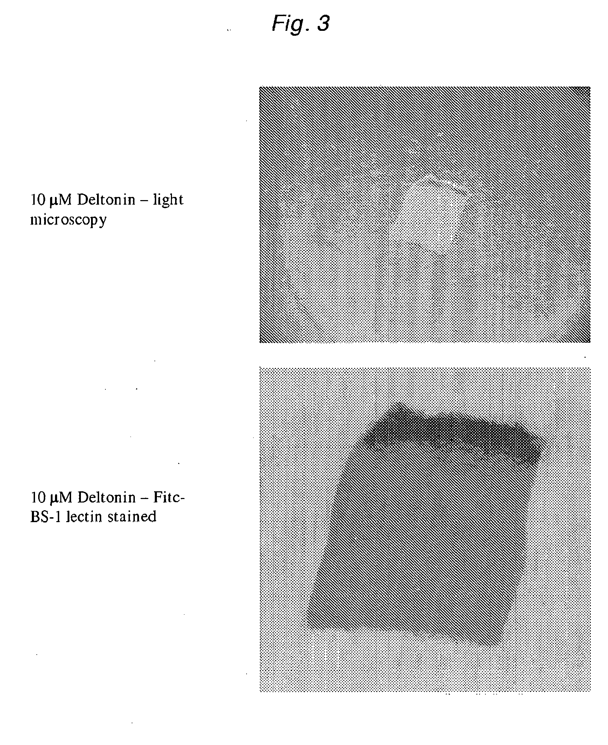

Representative examples of the samples are shown in Figures 2 and 3.

### Example 3

### Determination of inhibition of endothelial cell proliferation and migration by steroid saponins using the AngioSponge™ assay method

The AngioSponge™ assay enables slow release of agarose-captured growth factor, which stimulates endothelial cell proliferation and migration, and provides a tissue-like matrix for neovascularisation. The assay can be therefore be used to gauge the efficacy of treatments designed to inhibit angiogenesis, determined by counting blood vessels after CD31 immunohistochemical staining of endothelial cells (McCarty et al, International Journal of Oncology, 21:5-10, 2002).

Gelfoams (Pharmacia & Upjohn) were cut into approximately 7x7x7 mm pieces, under sterile conditions, and pre-soaked over night with sterile phosphate buffered saline. The hydrated gelfoams were then partially dried. Growth factor (bFGF) was diluted to an initial concentration of 2 µg/mL in warm (37°C) 0.4% agarose. The partially dried gelfoams were placed in the growth factor solution, or in warm 0.4% agarose without growth factor, and then transferred to a Petri dish containing warm 0.4% agarose (1:1 dilution, final growth factor concentration 1 µg/mL) for polymerisation and solidification.

70 female C3H/Hej mice were microchipped, weighed and randomised based on body weight into 7 groups with 10 mice per group. For implantation, the mice were anaesthetised by halothane inhalation. A small channel forged with a trocar needle between the 3rd and 4th mammary gland, the sponge was inserted to the full depth of the channel and the incision closed with a wound clip.

The compounds were formulated in N-methylpyrrolidone (NMP):PEG300:Water (1:9:10, v/v). Dosing was established on the basis *of in vitro* IC₅₀ data and preliminary toxicity studies. Compounds were administered daily by intraperitoneal injection, at a dosing volume of 10 mL/kg, once daily for the study period as shown in Table 5.

**Table 5**

| Treatment | Once daily dosing |
|---|---|
| Vehicle | NMP:PEG300:Water (1:9:10, v/v) |
| Vehicle + bFGF | NMP:PEG300:Water (1:9:10, v/v) |
| Dioscin + bFGF | 10 mg/kg |
| Deltonin + bFGF | 10 mg/kg |
| Trillin + bFGF | 60 mg/kg |
| Prosapogenin A + bFGF | 20 mg/kg |
| Sorafenib + bFGF | 60 mg/kg |

Treatment was started when all the animals had recovered fully from anaesthesia, and continued for 10 days. On Day 11, the angiosponges were excised and snap frozen in liquid nitrogen with OCT for CD31 staining on frozen sections.

CD31/PECAM-1 is an antigen present specifically on endothelial cells and can therefore be used as a marker for blood vessels. Frozen samples of the sponge were cryo-sectioned (12 µm), mounted on positively-charged Plus slides (Fisher Scientific), and air-dried for 30 minutes. Frozen sections were in turn fixed in cold acetone, acetone/chloroform (1:1, v/v), and acetone for 5 min each, and then washed with PBS (phosphate buffer saline). After blocking with PBS containing 5% normal horse serum and 1% normal goat serum, sections were incubated with PECAM-1 monoclonal rat anti-mouse CD31 antibody (1:5000 in blocking solution, PharMingen, San Diego, CA) for 4 hours at room temperature. The slides were then washed three times with PBS and incubated with adequate secondary anti-rat antibody. Positive reactions were visualized by incubating the slides in stable 3,3'-diaminobenzidine for 5-10 min. The sections were rinsed with distilled water, counter-stained with Gill's haematoxylin for 1 min, and mounted with Universal Mount (Research Genetics). Control samples exposed to secondary antibody alone showed no specific staining. Mouse placenta was used as a positive control. For quantification, 3 independent visual fields were chosen and positive stained spots or vessels with lumen were counted.

The results were collated as average number of blood vessels (CD31 counts) and are summarised in Table 6:

**Table 6**

| Treatment | Average number of blood vessels | Percent Induction |
|---|---|---|
| Vehicle | 0 | 0 |
| Vehicle + bFGF | 72 | 100 |
| Dioscin + bFGF | 35 | 49 |
| Deltonin + bFGF | 44 | 61 |
| Trillin + bFGF | 41 | 57 |
| Prosapogenin A + bFGF | 21 | 29 |
| Sorafenib + bFGF | 2 | 3 |

The Percent Induction for a treatment is the difference in CD31 counts between the treatment (+bFGF) and the vehicle alone (no bFGF), divided by the difference in CD31 counts between the vehicle with bFGF and the vehicle alone (no bFGF), expressed as a percentage.

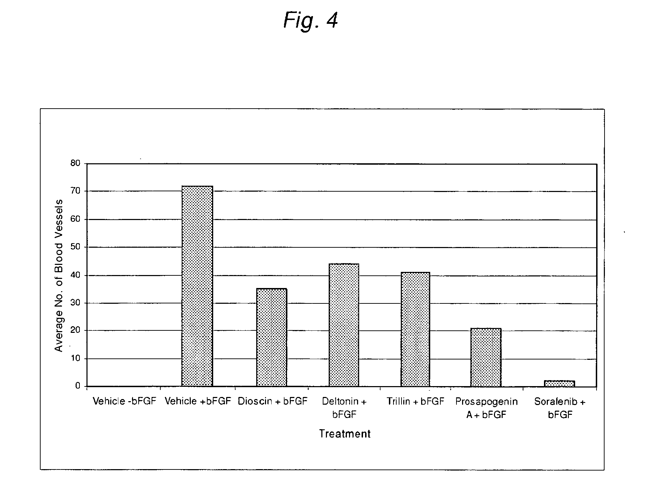

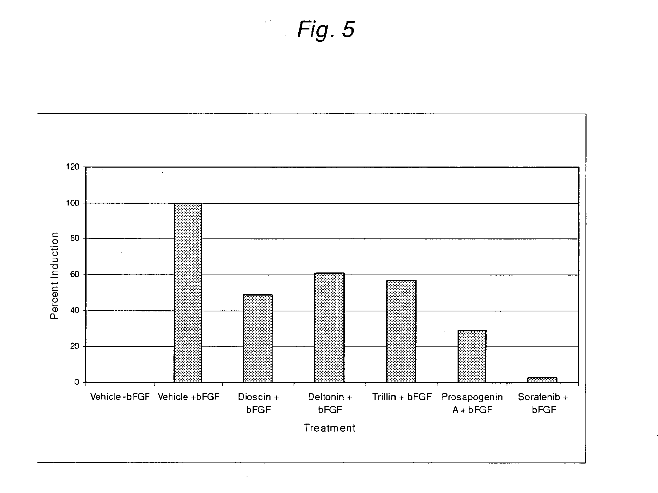

The number of blood vessels and percent induction are plotted graphically against treatments in Figures 4 and 5. The results demonstrate that steroid saponins cause a reduction in angiogenesis in the angiosponge model system.

### Example 3

### Determination of inhibition of endothelial cell proliferation and migration by steroidal saponins using the AngioChamber™ assay method

The AngioChamber™ assay utilises the normal physiological process of wound healing, which promotes the formation of a fibrous capsule around an implanted chamber (Wood et al, (2000) Cancer Research, 60(8):2178-89). The inclusion of bFGF in the chamber induces blood vessel development in the fibrous capsule. The assay therefore assesses the efficacy of treatments by gauging their effect both on the fibrous capsule formation, measured by the wet weight of the capsule at the termination of the study and by determination of blood vessel supply to the chamber by assaying for haemoglobin content. The haemoglobin content of the fibrous capsule is a measure of neovascularisation, which is assayed by the Drabkin Assay.

The angiochambers were porous tissue chambers made of perfluoro-alkoxy-Teflon and filled with 0.8% agar containing 20 IU/mL heparin, with or without 1 µg/mL human bFGF. Both ends of the chamber were sealed with removable caps of the same material. The chambers were filled under sterile conditions with 0.8% agar containing 20 IU/mL heparin, with or without 1 µg/mL human bFGF. The agarose solution was maintained at 37°C prior to filling the chambers.

70 female FvB mice were microchipped, weighed and randomised based on body weight into 7 groups with 10 mice per group. For implantation, the mice were anaesthetised by halothane inhalation. Approximately 150 µL sterile filtered air was injected subcutaneously in the back of each mouse, between the shoulder blades, resulting in an air pocket in which a small incision was made and the chamber inserted. The wound was closed with two 1.4 mm wound clips.

The treatments, which were all formulated in NMP:PEG300:Water (1:9:10, v/v) and administered daily by intraperitoneal injection, were as given in the Table 7:

**Table 7**

| Treatment | Once daily dosing |
|---|---|
| Vehicle | NMP:PEG300:Water (1:9:10, v/v) |
| Vehicle + bFGF | NMP:PEG300:Water (1:9:10, v/v) |
| Dioscin + bFGF | 10 mg/kg |
| Deltonin + bFGF | 10 mg/kg |
| Trillin + bFGF | 60 mg/kg |
| Prosapogenin A + bFGF | 20 mg/kg |
| Sorafenib + bFGF | 60 mg/kg |

Treatment was started when all the animals had recovered fully from anaesthesia, and continued for 5 days. On Day 6, the vascularised fibrous chambers were removed and the wet weight recorded for each implant. The chamber from each mouse was then snap frozen with liquid nitrogen and stored at -20°C until assessed for haemoglobin content.

The fibrous capsule samples were thawed and kept on ice during the procedure. Sterile water was added to each thawed sample, and the sample homogenised with an UltraTourax at high speed. To avoid cross-contamination, the UltraTourax was flushed for 30 seconds with 5 mL distilled water after homogenising each sample. The homogenate was transferred into 2 mL Eppendorf tubes and stored on ice. The Eppendorf tubes were centrifuged at 4°C and high speed (14,000 relative centrifugal force (RCF)) for 60 min. 1.0-1.5 mL of the intermediate aqueous solution (homogenate), between the pellet and the fat layer, was then transferred to fresh labelled 1.5 mL Eppendorf tubes and stored on ice. 50 µL of each homogenate was transferred into separate wells of a 96-well plate. 50 µL of water was transferred into 2 wells as blanks. 50 µL of Drabkin's reagent was then added to each well and mixed. After 15 min incubation at room temperature, the absorbance of each sample at 540 nm was measured and the volume of blood for each sample calculated from a standard curve.

The results were collated as average chamber weight, average blood content (from haemoglobin content, and Percent Induction, and are summarised in Table 8:

**Table 8**

| Treatment | Average chamber weight (g) | Average blood content (µL) | Percent Induction |
|---|---|---|---|
| Vehicle | 0.066 | 0.44 | 0 |
| Vehicle + bFGF | 0.375 | 2.21 | 100 |
| Dioscin + bFGF | 0.131 | 1.24 | 45 |
| Deltonin + bFGF | 0.126 | 0.79 | 20 |
| Trillin + bFGF | 0.421 | 1.46 | 58 |
| Prosapogenin A + bFGF | 0.389 | 1.92 | 84 |
| Sorafenib + bFGF | 0.322 | 0.92 | 27 |

The Percent Induction for a treatment is the difference in blood content between the treatment (+bFGF) and the vehicle alone (no bFGF), divided by the difference in blood content between the vehicle with bFGF and the vehicle alone (no bFGF), expressed as a percentage.

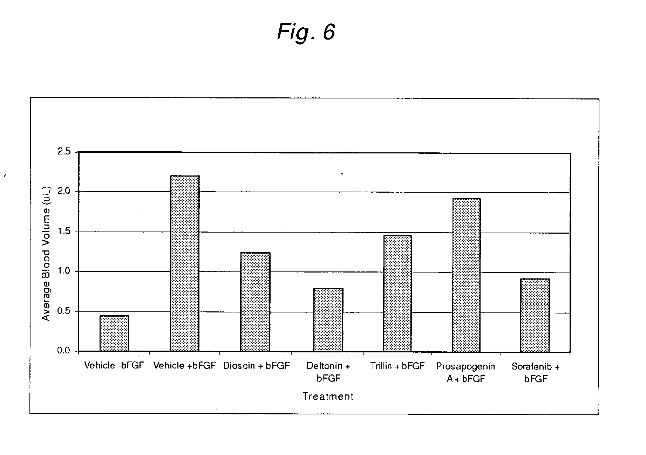

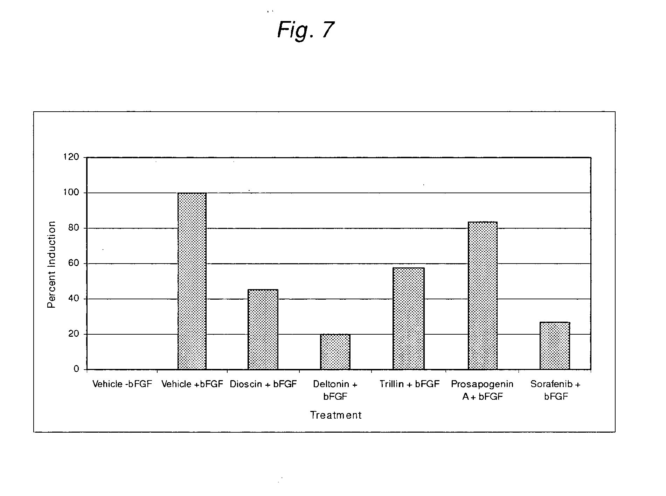

The average blood content of the angiochambers and percent induction are plotted graphically against treatments in Figures 6 and 7. The results demonstrate that steroid saponins cause a reduction in angiogenesis in the angiosponge model system.

## Claims

1. A steroid saponin for use in inhibiting angiogenesis in a human or animal subject, wherein the steroid saponin is selected from the group consisting of deltonin (diosgenin Rha2, [Glc4], Glc), dioscin (diosgenin Rha2, [Rha4], Glc), and prosapogenin A (diosgenin Rha2, Glc), and wherein the angiogenesis is angiogenesis associated with angiofibroma, corneal neovascularisation, retinal/choroidal neovascularization, diabetic retinopathy, age-related macular degeneration, arteriovenous malformations, arthritis, rheumatoid arthritis, osteoarthritis, psoriatic arthritis, lupus, connective tissue disorders, Osler-Weber syndrome, atherosclerotic plaques, psoriasis, pyogenic granuloma, retrolental fibroplasias, scleroderma, hemangioma; trachoma, haemophilic joints, hypertrophic scars, diseases or conditions associated with acute or chronic inflammation, diseases or conditions associated with chronic inflammation of the lung including asthma, sarcoidosis, inflammatory bowel diseases, Crohn's disease or ulcerative colitis.

2. A steroid saponin for use according to claim 1, wherein the steroid saponin reduces the amount of an anti-angiogenic agent required to be administered to the biological system to achieve a desired level of inhibition of angiogenesis.

3. A steroid saponin for use according to claim 1 or claim 2, wherein the anti-angiogenic agent is one or more agents selected from the group consisting of an anti-VEGF antibody, including a humanized and/or chimeric antibody, an anti-VEGF aptamer, a VEGF antisense oligonucleotide, angiostatin, endostatin, an interferon, interleukin 1, interleukin 12, retinoic acid, and a tissue inhibitor of metalloproteinase-2 and -9.

## Patentansprüche

1. Steroides Saponin zur Verwendung beim Inhibieren von Angiogenese bei einem Menschen oder Tier, wobei das steroide Saponin aus der Gruppe ausgewählt ist, die aus Deltonin (Diosgenin Rha2, [Glc4], Glc), Dioscin (Diosgenin Rha2, [Rha4], Glc) und Prosapogenin A (Diosgenin Rha2, Glc) besteht, und wobei die Angiogenese eine Angiogenese ist, die mit Angiofibrom, kornealer Neovaskularisation, retinaler/choroidaler Neovaskularisation, diabetischer Retinopathie, altersbedingter Makuladegeneration, arteriovenösen Missbildungen, Arthritis, rheumatoider Arthritis, Osteoarthritis, psoriatischer Arthritis, Lupus, Bindegewebestörungen, Osler-Weber-Syndrom, atherosklerotischen Plaques, Psoriasis, pyogenem Granulom, retrolentalen Fibroplasien, Sklerodermie, Hämangiom; Trachom, hämophilen Gelenken, hypertrophen Narben, Erkrankungen oder Zuständen, die mit akuter oder chronischer Entzündung verbunden sind, Erkrankungen oder Zuständen, die mit chronischer Entzündung der Lunge verbunden sind, einschließlich Asthma, Sarkoidose, entzündlichen Darmerkrankungen, Morbus Crohn oder Colitis ulcerosa einhergeht.

2. Steroides Saponin zur Verwendung nach Anspruch 1, wobei das steroide Saponin die Menge eines anti-angiogenen Mittels verringert, das an das biologische System verabreicht werden muss, um ein gewünschtes Niveau an Inhibierung der Angiogenese zu erreichen.

3. Steroides Saponin zur Verwendung nach Anspruch 1 oder Anspruch 2, wobei das anti-angiogene Mittel ein oder mehrere Mittel ist, die aus der Gruppe ausgewählt sind, die aus einem Anti-VEGF-Antikörper, einschließlich eines humanisierten und/oder chimären Antikörpers, einem Anti-VEGF-Aptamer, einem VEGF-Antisense-Oligonukleotid, Angiostatin, Endostatin, einem Interferon, Interleukin 1, Interleukin 12, Retinsäure und einem Gewebeinhibitor von Metalloproteinase-2 und -9 besteht.

## Revendications

1. Saponine stéroïde pour une utilisation dans l'inhibition de l'angiogénèse chez un sujet humain ou animal, la saponine stéroïde étant choisie parmi le groupe constitué de la deltonine (diosgénine Rha2, [Glc4], Glc), la dioscine (diosgénine Rha2, [Rha4], Glc) et la prosapogénine (diosgénine Rha2, Glc), et dans laquelle l'angiogénèse est une angiogénèse associée à un angiofibrome, une néovascularisation cornéenne, une néovascularisation rétinienne/coroïdienne, une rétinopathie diabétique, une dégénération maculaire liée à l'âge, des malformations artérioveineuses, de l'arthrite, de l'arthrite rhumatoïde, de l'ostéoarthrite, de l'arthrite psoriasique, un lupus, des troubles du tissu conjonctif, un syndrome d'Osler-Weber, des plaques d'athérosclérose, un psoriasis, un granulome pyogène, des fibroplasies rétrolentales, une sclérodermie, un hémangiome, un trachome, des articulations hémophiles, des cicatrices hypertrophiées, des maladies ou états associés à une inflammation aiguë ou chronique, des maladies ou états associés à une inflammation chronique du poumon incluant l'asthme, une sarcoïdose, des maladies inflammatoires de l'intestin, la maladie de Crohn ou une colite ulcéreuse.

2. Saponine stéroïde pour une utilisation selon la revendication 1, la saponine stéroïde réduisant la quantité d'un agent anti-angiogénique requis à administrer au système biologique pour obtenir un niveau souhaité d'inhibition de l'angiogénèse.

3. Saponine stéroïde pour une utilisation selon la revendication 1 ou la revendication 2, dans laquelle l'agent anti-angiogène est un ou plusieurs agents choisis parmi le groupe constitué d'un anticorps anti-VEGF, incluant un anticorps humanisé et/ou chimérique, un aptamère anti-VEGF, un oligonucléotide VEGF antisens, une angiostatine, une endostatine, un interféron, une interleukine 1, une interleukine 12, l'acide rétinoïque, et un inhibiteur de tissu des métalloprotéinases-2 et -9.
